(19) Europäisches Patentamt
European Patent Office
Office européen des brevets

(11) Publication number: **0 018 118**
**B1**

(12) # EUROPEAN PATENT SPECIFICATION

(45) Date of publication of patent specification: **22.06.83**

(51) Int. Cl.³: **C 07 C 148/00**

(21) Application number: **80300999.2**

(22) Date of filing: **31.03.80**

(54) **Production of thiophenols.**

(30) Priority: **02.04.79 US 26139**

(43) Date of publication of application:
**29.10.80 Bulletin 80/22**

(45) Publication of the grant of the patent:
**22.06.83 Bulletin 83/25**

(84) Designated Contracting States:
**AT BE CH DE FR GB IT NL SE**

(56) References cited:
**US - A - 2 402 686**
**US - A - 2 490 257**
**US - A - 3 994 980**

(73) Proprietor: **STAUFFER CHEMICAL COMPANY**
**Westport Connecticut 06880 (US)**

(72) Inventor: **Skrzec, Adam Edward**
**241 Germonds Road**
**West Nyack New York 10994 (US)**

(74) Representative: **Smith, Sydney et al,**
**Elkington and Fife High Holborn House 52/54**
**High Holborn**
**London WC1V 6SH (GB)**

**EP 0 018 118 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Production of thiophenols

This invention relates to a process for the production of a thiophenol, and more particularly to the production of a thiophenol by the vapor phase reaction of hydrogen sulfide and a halogenated aromatic compound to produce high yields of thiophenol with low quantities of byproduct aromatic thioethers and particularly diphenyl disulfide.

Thiophenols are useful as additives to lubricating oils, as intermediates in organic synthesis, as insecticides, fungicides and parasiticides and as ingredients of insecticidal, fungicidal and parasiticidal compositions, and they are useful in the preparation of synthetic resins, rubber, vulcanization accelerators, and the like.

U.S. Patent 2,490,257 to *Crowley et al.* describes the production of thiophenols by the vapor phase reaction of chlorobenzene and hydrogen sulfide in the presence of a catalyst, such as wood charcoal or alumina.

Japanese Patent 1970—19046 describes the production of thiophenols by the vapor phase reaction of chlorobenzene with hydrogen sulfide in the presence of a catalyst of a sulfide of Cu, Ag, Zn, Cd, Pd, Bi, Co or Mo supported on activated carbon.

Both of these processes suffer from low yields of thiophenol due to the fact that byproduct aromatic thioethers, such as diphenyl sulfide, and diphenyl disulfide are produced. Attempts to reduce the amounts of such byproducts and increase thiophenol yields have been made. For example, U.S. Patent 3,799,989 to *Sherk et al.* describes the production of thiophenol by the vapor phase reaction of chlorobenzene and hydrogen sulfide in a reaction zone containing a non-catalytic filler. The byproduct diphenyl sulfide formed during the reaction is subsequently converted to additional quantities of thiophenol by separating the diphenyl sulfide from the product stream and returning it to the reaction zone for cleavage to thiophenol in the presence of the reactant hydrogen sulfide. Japanese Patent 1971—8293 also describes the vapor phase reaction of chlorobenzene with hydrogen sulfide, but in the presence of a catalyst, such as activated charcoal or alumina, and also recycles the diphenyl sulfide to the reaction zone.

The aforementioned processes for increasing the yield of thiophenol and reducing the quantity of byproducts, such as aromatic thioethers, are not ideal for they involve the complex and costly separation of the byproduct diphenyl sulfide from the thiophenol, the constant recycling of such products, and such processes do not convert the byproduct diphenyl disulfide to thiophenol.

Attempts have been made to convert the byproduct diphenyl disulfide to thiophenol but such attempts generally require additional process equipment and result in disposal problems for other byproducts.

It is also known in the art from U.S. Patent 2,402,686 to *Signaigo* that aromatic thioethers in the presence of a sulphactive hydrogenation catalyst, e.g. sulphides of iron, nickel, cobalt, copper, etc. may be hydrogenated to thiophenol. *Signaigo* indicates that it is preferred to use hydrogen gas in substantially pure form as the reducing agent, however, hydrogen admixed with hydrogen sulfide may also be used. Additionally of interest is the fact that U.S. Patent 2,506,416 to *Gilbert et al.* describes the reduction of a diaryl polysulfide, i.e. diphenyl disulphide, by heating in the presence of a petroleum hydrocarbon to produce the thiophenol; and U.S. Patent 3,994,980 to *Kubicek* describes a process for the preparation of a mercaptan by reacting an organic compound containing a carbonyl group with a sulfur source such as hydrogen sulfide in the presence of a specific catalyst. *Kubicek* states that "more satisfactory results are achieved when hydrogen is also used". None of these references, however, teach or suggest reacting hydrogen sulfide and a source of hydrogen with a halogenated aromatic compound, particularly chlorobenzene, preferably under vapor phase conditions, to produce a thiophenol.

Accordingly, it is an object of this invention to provide a process for the conversion of a halogenated aromatic compound, e.g. chlorobenzene, to a thiophenol, specifically thiophenol, in high yields and with low quantities of byproduct aromatic thioethers, and diphenyl disulfide.

It is a further object of this invention to considerably reduce the cost and problems associated with the separation of byproduct aromatic thioethers, e.g. diphenyl sulfide, and diphenyl disulfide, from the product stream produced from the reaction of a halogenated aromatic compound with hydrogen sulfide.

It is a further object of this invention to provide a process for the production of thiophenols which can be conducted in a batch or continuous manner.

In a first embodiment, the present invention relates to a process for producing a thiophenol by reacting a halogenated aromatic compound with hydrogen sulfide, characterised in that the reaction is carried out in the presence of a source of hydrogen, whereby the yield of thiophenol is increased and the quantity of byproduct aromatic thioethers and diphenyl disulfide is decreased.

In a second embodiment, the present invention relates to a process for producing a thiophenol by reacting a halogenated aromatic compound with hydrogen sulfide characterised in that one increases the temperature as the total conversion decreases, whereby the total conversion is increased.

The terms "halogenated aromatic compound" and "source of hydrogen" which will be understood by those skilled in the art are further clarified below.

According to the present invention, hydrogen sulfide and a source of hydrogen are reacted with a halogenated aromatic compound to produce a thiophenol. The thiophenol is produced in high yields with low quantities of byproduct aromatic thioethers, and diphenyl disulfide.

The process of this invention, broadly, is an improvement over well known processes for producing a thiophenol by the reaction of a halogenated aromatic compound with hydrogen sulfide. The process of this invention may be accomplished with the same reactants, i.e. halogenated aromatic compounds, under the same conditions of reaction, e.g. temperature, pressure, etc., using the same catalysts to produce the same thiophenols as these well known processes. Preferably, the process of this invention is a vapor phase reaction process.

The process of this invention may also be utilized in conjunction with any of the other well known processes which recycle the byproduct impurities, e.g. byproduct aromatic thioethers, see for example, U.S. Patent 3,799,989 to *Sherk et al.* and Japanese Patent 1971—8293. The entire disclosures of both these references are incorporated herein by reference. Ideally, the process of this invention may completely eliminate the necessity of recycling such byproducts, but this is highly dependent upon the hydrogen source, the amount of hydrogen produced, the specific reaction conditions, and the apparatus utilized. Thus, in its broadest, form the process of this invention will reduce the quality of byproduct aromatic thioethers which are recycled, and in its optimum form completely eliminate the necessity for recycling such products.

While this invention will be described primarily with respect to the formation of thiophenol from chlorobenzene and hydrogen sulfide, the halogenated aromatic compound may include one or more alkyl or other non-reactive groups which are stable during the reaction. For example, the alkyl group substituent on the aromatic ring may have from one to about five or more carbons. Also, in lieu of one or more alkyl substituents, other non-reactive substituents may be included, e.g. tolyl, xylyl and mesityl.

*Crowley et al.* exemplifies some of the thiophenols that may be produced and the corresponding halogenated aromatic compounds that may be utilized in the process of this invention. The halogenated aromatic compound may be selected from the group consisting of mono- and dihalogenated benzenes, mono- and dihalogenated mono- and polyalkyl benzenes in which one or more of the alkyl group has up to 24 to 26 carbon atoms, mono- and dihalogenated polynuclear aromatic compounds and mono- and dihalogenated mono- and polyalkyl polynuclear aromatic compounds in which one or more of the alkyl substituents has up to 24 to 26 carbon atoms. The reader is referred to *Crowley et al.,* column 4, line 30 through column 6, line 10, for a more definitive explanation of the halogenated aromatic compounds which may be utilized in this invention. The entire disclosure of U.S. Patent 2,490,257 to *Crowley et al.* is incorporated herein by reference.

Thus, the term "halogenated aromatic compound" includes all of the foregoing type compounds that one skilled in the art would utilize in this type reaction, as well as chlorobenzene.

By the use of the term "source of hydrogen" it is meant hydrogen or any composition when subjected to the conditions of the reaction release hydrogen which can be utilized in the reaction. An example of such a source of hydrogen is methane. It is preferred, however, to utilize hydrogen.

The process of this invention can proceed with or without a catalyst. It is preferred, however, to utilize the conventional catalysts, well known in the art, for this type process. *Crowley et al.,* for example, describes the use of a highly absorbent solid catalyst, such as activated alumina, silica, silica alumina, silica-alumina-iron gel and activated charcoal. Japanese Patent 1970—19046, the entire disclosure of which is incorporated herein by reference, describes the use of a sulfide of Cu, Ag, Zn, Cd, Pb, Bi, Co, or Mo supported on an activated carbon. The process of this invention may utilize any of these type catalysts. It is highly preferred, however, to utilize activated carbon.

The reaction, for a given yield, may be carried out in the presence of a catalyst at lower temperatures than those at which the reaction takes place in the abesnce of a catalyst. For example, temperatures of about 400°C. to about 600°C. have been found to be satisfactory for the catalyzed reaction.

A particularly preferred manner of operating the process of this invention is to increase the temperature as the total conversion decreases due to deactivation of the catalyst. This may be done in a gradual or stepwise manner. The rate of increase in the temperature is highly dependent on the catalyst, the halogenated aromatic compound used, the operating temperature, etc., but can readily be determined by simple experimentation by one skilled in the art. It has been found that about a 20°C. increase in temperature for a drop of about 5% in yield has worked effectively to increase the yield by about 5%. This process for increasing the temperature as the total conversion decreases due to deactivation of the catalyst may also be used in the broad process of reacting a halogenated aromatic compound with hydrogen sulfide, i.e. with or without a catalyst or hydrogen.

The reaction can be carried out at superatmospheric, atmospheric or sub-atmospheric pressures.

The reactants are passed through a heated reaction zone, preferably concurrently. Normally the reactants will be preheated before being fed to the reaction zone. The feed is arranged so that there is efficient mixing of the reactants in a very short time, i.e. several seconds. The liquid product, upon condensation, consists of the thiophenol and the unreacted halogenated aromatic compound. Any byproduct aromatic thioethers produced may be recycled with the unreacted halogenated aromatic compound in the conventional manner, i.e. as described in *Scherk et al.* and Japanese Patent

1971—8293.

The hydrogen sulfide, source of hydrogen and halogenated aromatic compound may be reacted in any proportion. It is preferred, however, to employ an amount of hydrogen sulfide in excess of that required to satisfy the following equation:

Formula 1

$$RCl + H_2S \rightarrow RSH + HCl$$

wherein "R" is an aromatic substituent.

That is to say, the hydrogen sulfide is preferably present in an amount in excess of equal molecular proportions. A mole ratio of hydrogen sulfide to halogenated aromatic compound of about 1:1 to about 10:1 will produce satisfactory results, although higher or lower molar ratios may be used if desired. Mole ratios greater than about 2:1 are preferred, with about 3:1 to about 5:1 having been found effective in producing substantial yields of thiophenol.

The mole ratio of hydrogen (produced from the hydrogen source) to halogenated aromatic compound is not critical. It is preferred, however, to have present an amount of hydrogen which produces an increase in yield of thiophenol over that which would be produced without the hydrogen present. A preferred mole ratio of hydrogen to halotenated aromatic compound is from about .05:1 to about 3:1, with about .05:1 to about .5:1 being highly preferred.

It is also within the scope of this invention to use a greater quantity of either the hydrogen sulfide, the source of hydrogen, e.g. hydrogen, or both should conditions of temperature, pressure, conversion or product distribution so require. In general, however, the amounts expressed above should be used.

The hydrogen sulfide, hydrogen source and halogenated aromatic compound may be passed over the catalyst at any convenient rate, depending upon the temperature, pressure, composition of the reactants and the products desired. A feed rate of hydrogen sulfide, hydrogen, and halogenated aromatic compound of from about 1 to about 80 moles per liter of catalyst per hour will generally produce suitable results, although higher or lower rates may be used. A preferred feed rate is from about 5 to about 15 moles per liter of catalyst per hour.

The reaction may be allowed to take place for any suitable length of time. The duration of the run, i.e. residence time within the reaction zone, will depend upon feed rate, the reactant mixture utilized, temperature, pressure, catalyst used, and products desired. Residence times of from about 5 to about 60 seconds are generally suitable although longer or shorter runs may be desirable in some cases.

The products of reaction, i.e. thiophenol, any byproduct aromatic thioethers and any unreacted reactant mixture, can be recovered after one pass through the reaction or may be recycled.

The invention may be executed in any suitable type of apparatus and the process may be carried out in a batch-wise, intermittent or continuous manner, although a continuous process is preferred.

The separation of the products of reaction from each other, i.e. the thiophenol from any byproducts and unreacted reactant mixture, may be accomplished by processes well known in the art, see for example, *Sherk et al.* and U.S. Patent 4,024,191 to *Scoggin.*

The reactions occurring within the reaction zone are believed to be as follows:

Formula 1

$$RCl + H_2S \rightarrow RSH + HCl$$

Formula 2

$$H_2S \rightleftarrows H. + .SH \rightleftarrows H_2 + S$$

Formula 3

$$RCl + H_2 \rightarrow R + HCl$$

Formula 4

$$RCl + RSH \rightarrow R_2S + HCl$$

Formula 5

$$3\ RSH + HCl \rightarrow RCl + R_2S + 2H_2S$$

Formula 6

$R_2S + S \rightarrow R{-}S{-}S{-}R$

Formula 7

$R{-}S{-}S{-}R + H_2 \rightarrow 2RSH$

Formula 8

$R{-}S{-}R + H_2 \rightarrow RSH + R$

It is believed that the process of this invention produces the high yields of thiophenol with low quantities of byproducts e.g. aromatic thioethers and diphenyl disulfide because the hydrogen in the reactant mixture either (a) prevents the formation of byproduct aromatic thioethers, i.e. Formulas 1 and 2 and/or (b) cleaves the byproduct aromatic thioethers and diphenyl sulfide to thiophenol, i.e. Formulas 7 and 8.

The following examples serve to illustrate the invention and its advantages.

Example 1

A one inch (2.54 cm.) I.D. by 5 1/2 foot (1.67 meters) high catalytic bed reactor was used for this test. The catalyst bed was 423 grams (5 feet — 1.52 meters — in height), 3.08 liters of PPG (6 x 16) activated carbon. The bed was brought to a temperature of 450°C. by passing heated nitrogen therethrough.

Monochlorobenzene was uniformly fed to the catalyst bed at a rate of 71 grams (0.63 moles) per hour.

The monochlorobenzene was vaporized and superheated to 400°C. prior to entry into the catalytic bed.

Hydrogen sulfide was uniformly fed to the catalyst bed at a rate of 64 grams (1.89 moles) per hour. The hydrogen sulfide was prehated to to 400°C. prior to entry into the catalytic bed. Hydrogen gas was added to the hydrogen sulfide stream prior to preheating at a rate of .38 grams (0.19 moles) per hour.

The mole ratio of hydrogen sulfide:monochlorobenzene:hydrogen was 3:1:0.3.

The reaction was allowed to proceed continuously for 300 hours. The following results were obtained:

TABLE I

HYDROGEN

Catalytic Bed Temp: 450°C.

1 atmosphere pressure

R = 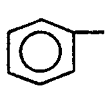

| Process Time (hrs.) | % Mono-chlorobenzene Converted to: RSH | $R_2S$ | $R_2S_2$ | Total Conversion Per Pass |
|---|---|---|---|---|
| 40 | 24.6 | 14.9 | 0 | 39.5 |
| 100 | 19.9 | 8.8 | 0 | 28.7 |
| 150 | 17.9 | 8.4 | 0 | 26.3 |
| 200 | 18.0 | 6.9 | Trace | 24.9 |
| 300 | 16.9 | 4.1 | Trace | 21.0 |

Comparative Examples

The exact same test was performed as Example 1, except without injection of hydrogen into the catalytic bed. The following results were obtained:

TABLE II

NO HYDROGEN

Catalytic Bed Temp: 450 °C

1 atmosphere pressure

R = 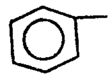

| Process Time | % Mono-chlorobenzene Converted to: RSH | $R_2S$ | $R_2S_2$ | Total Conversion Per Pass |
|---|---|---|---|---|
| 40 | 22 | 15.4 | 1.6 | 39 |
| 100 | 18 | 9.0 | 1.4 | 28.4 |
| 150 | 16.3 | 8.8 | 1.4 | 26.5 |
| 200 | 16.2 | 7.1 | 1.3 | 24.6 |
| 300 | 14.8 | 4.2 | 1.0 | 20.0 |

Example 2

This Example was performed in a bench scale hot tube static bed reaction system consisting of two (2) 1 inch (2.50 cms) by 6 foot long (1 m. 89 cms) schedule 40 pipes, constructed of 310 stainless steel and Incoloy 825 alloy. The gas distributor was a circular 316 stainless steel disk with 21—1/16 inch (15 mms) holes.

Heat was supplied by externally wound beaded nichrome wire heaters. The temperature of the bed was maintained constant by adjusting the power input to wire heaters. Each reactor was equipped with 5 thermocouples for recording and controlling temperatures. Reactants were fed through separate feed lines entering through the side of the wind box which promoted intimate contact of the preheated hydrogen sulfide and chlorobenzene before passing through the distributor plate and activated carbon bed.

Reactor off-gases were condensed in a two pass stainless steel condenser and collected in a glass receiver. The vent lines of both reactors had a valve arrangement that allowed condensate collection from both or individual reactors.

In one run lasting only 32 hours, methane was substituted for hydrogen as a source of hydrogen:

TABLE III

METHANE

Catalytic Bed Temp: 450°C.

1 atmosphere pressure

R = 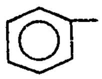

| Process Time | % Mono-chlorobenzene Converted to: | | | Total Conversion Per Pass |
|---|---|---|---|---|
| | RSH | $R_2S$ | $R_2S_2$ | |
| 6 | 44.3 | 8.1 | 0 | 52.4 |
| 15 | 38.6 | 7.4 | 0 | 46.0 |
| 20 | 32.2 | 7.6 | 0 | 39.8 |
| 25 | 30.2 | 7.5 | Tr | 37.7 |
| 30 | 28.1 | 7.1 | Tr | 35.2 |

Small amounts of methyl mercaptan appeared in the product which was probably produced according to the following formulas:

$$CH_4 \rightarrow CH_3 + H\cdot$$

$$H_2S \rightleftarrows H\cdot + \cdot SH$$

$$CH_3\cdot + \cdot SH \rightarrow CH_3SH$$

$$H\cdot + H\cdot \rightarrow H_2$$

## Example 3

The exact same test as Example 1 was performed except as indicated herein.

A fresh activated carbon catalyst bed was placed in the reactor. The reaction was initially conducted at 400°C. The reactor was divided into three heating zones of equal height. Each zone was composed of a nichrome resistance wire wound around the reactor. Since the reaction was only slightly exothermic, isothermal reaction conditions were maintained by adjusting the electrical power fed to each heating zone. It was found that by adjusting power input the temperature in the reaction zone could be maintained within $\pm$ 5°C.

With a fresh bed of activated carbon the initial total conversion per pass was about 41%. The total conversion then gradually decreased to about 20% over the next 240 hours. It had been found from past tests that if the reaction were allowed to proceed at 400°C. the total conversion would have been about 5% to 7% within an additional 60 hours, i.e. 300 hours total time. Total conversions below 14% are considered uneconomical.

The temperature of the bed was increased at 240 hours total time, to about 420°C. by increasing the power input to each heating zone. The total conversion increased over the next 16 hours to about 25%. After about 80 hours, the total conversion again dropped to 20%.

The temperature of the bed was then increased to 440%C. The total conversion subsequently increased to about 25%. After about 80 hours the total conversion again dropped to about 20%.

This process was repeated several more times until the temperature of the bed was 520°C. This temperature was determined to be the maximum temperature for conducting this reaction under these conditions.

**Claims**

1. A process for producing a thiophenol by reacting a halogenated aromatic compound with hydrogen sulphide, characterised in that the reaction is carried out in the presence of a source of hydrogen.

2. A process as claimed in claim 1 characterised in that the halogenated aromatic compound is chlorobenzene and the source of hydrogen is hydrogen.

3. A process as claimed in claim 1 or claim 2 characterised in that the reaction is carried out at a temperature of from 400°C to 600°C.

4. A process as claimed in claim 3 characterised in that the reaction is carried out at a temperature which is increased gradually or in a stepwise manner as the total conversion decreases.

5. A process for producing a thiophenol by reacting a halogenated aromatic compound with hydrogen sulphide characterised in that one increases the temperature as the total conversion decreases.

6. A process as claimed in claim 5 characterised in that the halogenated aromatic compound is chlorobenzene.

7. A process as claimed in claim 5 or claim 6 characterised in that the temperature is between 400°C and 600°C.

8. A process as claimed in any of claims 5 to 7 characterised in that the reaction is carried out in the presence of an effective catalyst for the reaction.

9. A process as claimed in any of claims 5 to 8 characterised in that one increases the temperature gradually to maintain the total conversion substantially constant.

10. A process as claimed in any of claims 5 to 8 characterised in that the temperature is increased in a stepwise manner.

**Revendications**

1. Un procédé de production d'un thiophénol par réaction d'un composé halo-aromatique avec l'acide sulfhydrique, caractérisé en ce que la réaction est effectuée en présence d'une source d'hydrogène.

2. Un procédé selon la revendication 1, caractérisé en ce que le composé halo-aromatique est le chlorobenzène et que la source d'hydrogène est l'hydrogène.

3. Un procédé selon la revendication 1 ou la revendication 2, caractérisé en ce que la réaction est effectuée à une température comprise entre 400 et 600°C.

4. Un procédé selon la revendication 3, caractérisé en ce que la réaction est effectuée à une température qui est augmentée graduellement ou par étape lorsque le taux de conversion décroît.

5. Un procédé de production d'un thiophénol par réaction d'un composé halo-aromatique avec l'acide sulfhydrique, caractérisé en ce qu'on augmente la température lorsque le taux de conversion total décroît.

6. Un procédé selon la revendication 5, caractérisé en ce que le composé halo-aromatique est le chlorobenzène.

7. Un procédé selon la revendication 5 ou la revendication 6, caractérisé en ce que la température est comprise entre 400 et 600°C.

8. Un procédé selon une quelconque des revendications 5 à 7, caractérisé en ce que la réaction est effectuée en présence d'un catalyseur efficace pour la réaction.

9. Un procédé selon une quelconque des revendications 5 à 8, caractérisé en ce qu'on augmente la température graduellement pour maintenir le taux de conversion total sutstantiellement constant.

10. Un procédé selon une quelconque des revendications 5 à 8, caractérisé en ce que la température est augmentée par étape.

**Patentansprüche**

1. Verfahren für die Herstellung eines Thiophenols durch Reaktion einer halogenierten, aromatischen Verbindung mit Schwefelwasserstoff, dadurch gekennzeichnet, daß die Reaktion in Gegenwart einer Wasserstoffquelle durchgeführt wird.

2. Verfahren nach Anspruch 1, dadurch gekennzeichnet, daß die halogenierte, aromatische Verbindung Chlorbenzol und die Wasserstoffquelle Wasserstoff ist.

3. Verfahren nach Anspruch 1 oder 2, dadurch gekennzeichnet, daß die Reaktion bei einer Temperatur von 400°C bis 600°C durchgeführt wird.

4. Verfahren nach Anspruch 3, dadurch gekennzeichnet, daß die Reaktion bei einer Temperatur durchgeführt wird, die graduell oder schrittweise erhöht wird, wenn die Gesamtumwandlung abnimmt.

5. Verfahren zur Herstellung eines Thiophenols durch Reaktion einer halogenierten, aromatischen Verbindung mit Schwefelwasserstoff, dadurch gekennzeichnet, daß man die Temperatur erhöht, wenn die Gesamtumwandlung abnimmt.

6. Verfahren nach Anspruch 5, dadurch gekennzeichnet, daß die halogenierte, aromatische Verbindung Chlorbenzol ist.

7. Verfahren nach Anspruch 5 oder 6, dadurch gekennzeichnet, daß die Temperatur zwischen 400 und 600°C liegt.

8. Verfahren nach einem der Ansprüche 5 bis 7, dadurch gekennzeichnet, daß die Reaktion in Gegenwart eines für die Reaktion wirksamen Katalysators durchgeführt wird.

9. Verfahren nach einem der Ansprüche 5 bis 8, dadurch gekennzeichnet, daß man die Temperatur graduell erhöht, um die Gesamtumwandlung im wesentlichen konstant zu halten.

10. Verfahren nach einem der Ansprüche 5 bis 8, dadurch gekennzeichnet, daß die Temperatur schrittweise erhöt wird.